# EUROPEAN PATENT SPECIFICATION

(11) **EP 2 362 787 B1**
(45) Date of publication and mention of the grant of the patent: **08.03.2017**
(21) Application number: 09748837.3
(22) Date of filing: 06.11.2009
(51) Int. Cl.: A61L 9/20, A61L 9/22, C02F 1/32, C02F 1/46, H01S 3/22, H01S 3/08

(54) **DECONTAMINATION APPARATUS AND METHOD**
DEKONTAMINATIONSGERÄT UND VERFAHREN
APPAREIL ET PROCÉDÉ DE DÉCONTAMINATION

(30) Priority: 06.11.2008 GB 0820358
(43) Date of publication of application: 07.09.2011
(73) Proprietor: Nviro Cleantech (Cayman Islands) Limited, London W1S 1HX (GB); University Court of The University of Glasgow, Glasgow G12 8QQ (GB)
(72) Inventor: FOSS-SMITH, Patrick, London W1S 1HX (GB); WATSON, Ian, Alistair, Glasgow G12 8QQ (GB)
(74) Representative: Naylor, Matthew John
(86) International application number: PCT/GB2009/002625
(87) International publication number: WO 2010/052473

(56) References cited:
- WO-A1-94/03794
- WO-A1-2005/094906
- GB-A- 2 340 368
- US-A- 4 676 639
- US-A1- 2003 103 204
- US-A1- 2006 207 431

## Description

The present invention relates to a decontamination apparatus and method.

Decontamination technologies are known for the substantial removal or deactivation of chemical agents, viruses, and/or bacteria from typically a fluid medium such as a liquid or gas, and more typically from water or air.

Conventionally, these technologies utilise the biocidal properties of ultraviolet (UV) light. A first property of UV light is that it ionises oxygen to produce ozone, which can kill many pathogens. A second property is that the UV radiation itself can directly disrupt surface tissue and/or DNA bonds in micro-organisms, thereby killing them, rendering them inert and/or preventing reproduction, and can alter the chemical properties of some fluid-borne contaminants.

In general the decontamination apparatus takes the form of a UV lamp such as a mercury-vapour lamp disposed to irradiate the potentially contaminated fluid (see for example the Wikipedia articles *Ultraviolet_germicidal_irradiation* and *Germicidal_lamp*).

However, it will be appreciated that the efficiency with which the light from any UV source can decontaminate a fluid is dependent at least in part upon the extent of exposure of the fluid to the UV light. For example, WO-A-01/60418 extends the exposure of the fluid to the UV source by placing the fluid in a reflective chamber, into which the UV light is then shone.

It will be similarly appreciated that the efficiency of decontamination can also depend upon the intensity of the UV light. It is therefore also desirable to increase the intensity of UV light incumbent upon the potentially contaminated fluid.

The present invention seeks to address, mitigate or alleviate the above problem.

In a first aspect of the present invention, there is provided a decontamination method as set out in claim 1.

In another aspect of the present invention, there is provided a decontamination method as set out in claim 8.

Advantageously, by passing the fluid for decontamination through the optical resonance cavity, it is exposed to much higher levels of radiation than those emitted through the output coupler, thereby improving the efficiency with which the laser can decontaminate the fluid.

Further respective aspects and features of the invention are defined in the appended claims.

Embodiments of the present invention will now be described by way of example with reference to the accompanying drawings, in which:
Figure 1 is a schematic diagram of a chamber and fluid-conditioning system known in the art.
Figure 2 is a schematic diagram of a fluid-conditioning system known in the art.
Figure 3 is a schematic diagram of a fluid-conditioning system comprising a decontamination apparatus in accordance with an embodiment of the present invention.
Figure 4 is a schematic diagram of a laser known in the art.
Figure 5 is a schematic diagram of a decontamination apparatus in accordance with an embodiment of the present invention.
Figure 6 is a schematic diagram of a decontamination apparatus in accordance with an embodiment of the present invention.
Figure 7 is a schematic diagram of a decontamination apparatus in accordance with an embodiment of the present invention.
Figure 8 is a schematic diagram of a decontamination apparatus in accordance with an embodiment of the present invention.
Figure 9 is a schematic diagram of a decontamination apparatus in accordance with an embodiment of the present invention.
Figure 10 is a schematic diagram of a decontamination apparatus in accordance with an embodiment of the present invention.
Figure 11 is a schematic diagram of a decontamination apparatus in accordance with an embodiment of the present invention.
Figures 12A and 12B are schematic diagrams of parallel and serial arrangements of decontamination apparatuses in accordance with an embodiment of the present invention.
Figure 13 is a schematic diagram of a plasma or electric field generator in accordance with an embodiment of the present invention.
Figure 14A is a schematic diagram of a roughened electrode in accordance with an embodiment of the present invention.
Figure 14B is a schematic diagram of a plasma or electric field generator comprising roughened electrodes in accordance with an embodiment of the present invention.
Figure 15A is a graph of surface roughness for different laser marking conditions in accordance with an embodiment of the present invention.
Figures 15B-D are images of a metal surface for different laser marking conditions in accordance with an embodiment of the present invention.
Figure 16 is a schematic diagram of a roughened electrode in accordance with an embodiment of the present invention.
Figures 17A and 17B are schematic diagrams of a plasma or electric field generator comprising rotatable electrodes in accordance with an embodiment of the present invention.
Figure 18 is a flow diagram of a method of fluid decontamination in accordance with an embodiment of the present invention.

A decontamination apparatus and method are disclosed. In the following description, a number of specific details are presented in order to provide a thorough understanding of the embodiments of the present invention. It will be apparent, however, to a person skilled in the art that these specific details need not be employed to practise the present invention. Conversely, specific details known to the person skilled in the art are omitted for the purposes of clarity where appropriate.

In an example embodiment of the present invention, an air conditioning system comprises a pump (e.g. a fan), a temperature modulator and a filter. In addition, the air conditioning system has been adapted to comprise a decontamination apparatus. The decontamination apparatus includes a laser comprising in turn two mirrors, being a first high reflector and an output coupler (or alternatively a second high reflector), between them forming a so-called optical cavity or resonator cavity within which a standing light wave can form. Within the resonator cavity is axially disposed a gain medium for generating laser light and substantially axially adjacent thereto is at least a first channel, substantially optically transparent in the emission frequency of the laser, through which the air for decontamination flows. By passing the air through at least a first portion of the resonator of the laser in this fashion, the air is exposed to much greater levels of radiation (potentially 100 times greater) than would be the case if the air was irradiated in a more conventional fashion by laser light emitted from an output coupler. Consequently, the intensity of light (whether UV in the case of for example an Excimer laser, or of some other frequency) is significantly increased with a corresponding increase in the effectiveness of decontamination. Advantageously this provides for comparatively greater levels of decontamination and/or air flow through the decontamination apparatus.

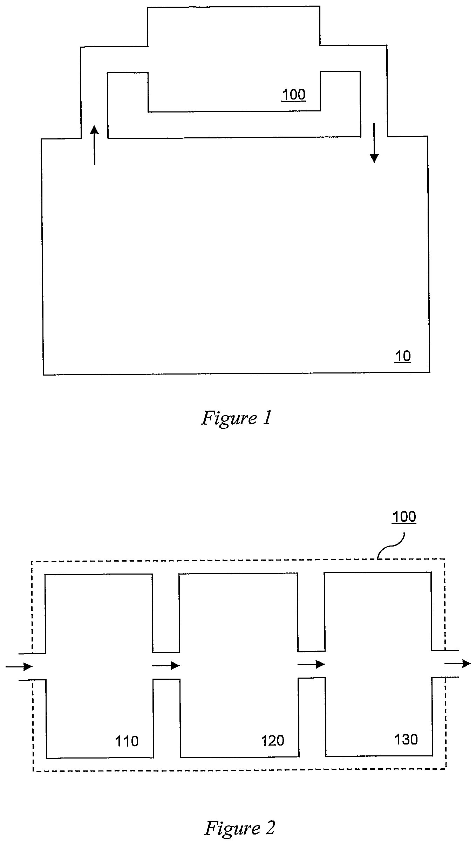

Referring now to Figures 1 and 2 a chamber 10 is serviced by a fluid conditioning unit 100. The chamber is filled with a fluid, typically either air or water but potentially some other gas or liquid. The conditioning unit typically comprises a pump 110 to circulate the fluid, a temperature modulation unit 120 (such as a refrigeration unit), and a filter 130. It will be appreciated that the order of pump, modulation unit and filter may vary. Optionally the system may also include a dehumidifier (not shown).

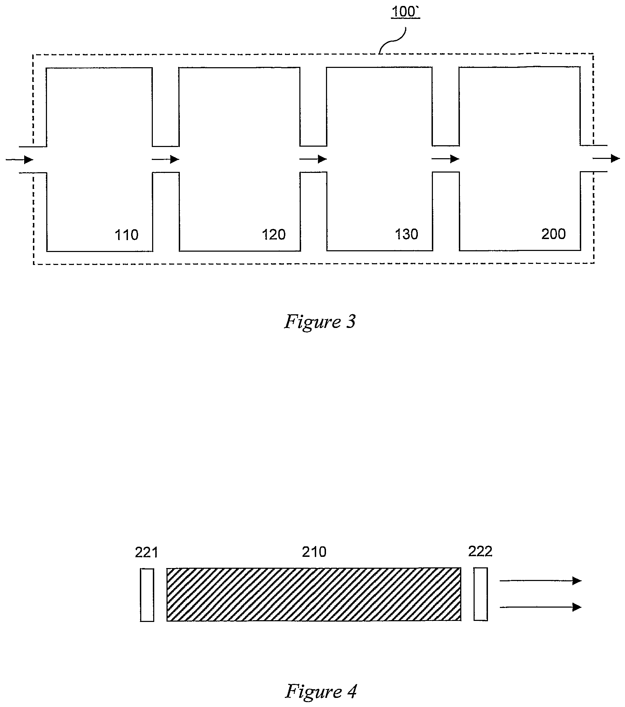

Referring now to Figure 3 in an embodiment of the present invention an adapted conditioning unit 100' further comprises a decontamination apparatus 200 preferably positioned at a point downstream of filter 130 and, potentially, the dehumidifier (not shown).
Such a decontamination apparatus may comprise a laser as the ultraviolet light source. Referring now to Figure 4, a known UV laser comprises a high reflector 221, an output coupler (a partial reflector) 222, and a lasing gain medium 210, such an Excimer. Upon receiving sufficient energy to achieve a population inversion (wherein a majority of atoms/molecules within the gain medium have reached an exited state), the laser emits a pulse or beam of UV light through the output coupler 222.

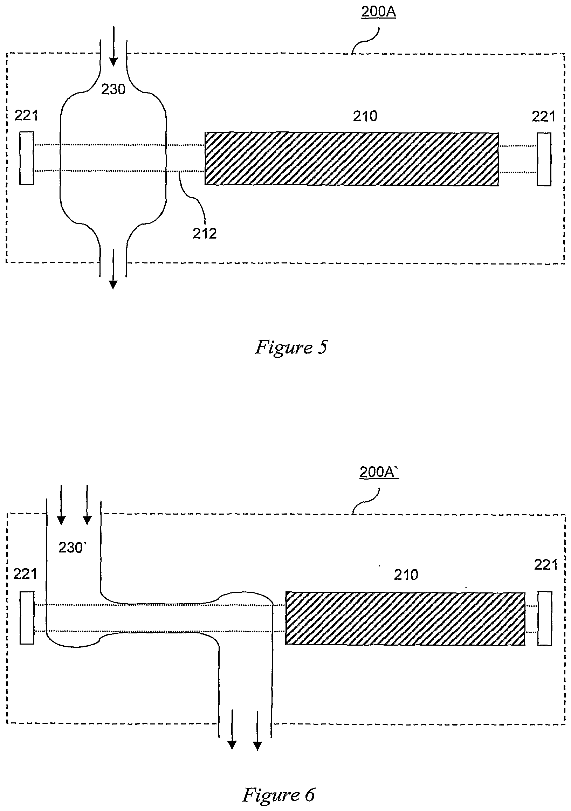

Referring now also to Figure 5, in a first instance a decontamination apparatus 200A comprises a laser with first high reflector 221 and a second high reflector 221 axially spaced apart from the first. Optionally the second high reflector may be an output coupler 222 (as seen in Figure 4), for example to enable confirmation of lasing action by external inspection. Between them, the reflectors form a so-called optical resonator (or simply 'resonator'), within which light 212 of a particular frequency can form a standing wave. This light is generated by a gain medium 210, suitably energised (or 'pumped') by an appropriate energy pump (not shown). Possible pumps known in the art include a flash lamp, a further laser, or an electric current.

The choice of gain medium will depend upon the required quality of light. For biocidal applications short wave UV light is desirable and suitable gain media include some Excimers, such as Krypton-Fluorine, which emits light at 248nm. For other properties, other frequencies of light may be required and a corresponding gain medium selected. For example carbon dioxide may be chosen for infra-red light.

Disposed between the gain medium and one of the reflectors, a channel 230 passes through the laser resonator, within which the fluid for decontamination - for example air - flows. Consequently the fluid passes through at least a first portion of the laser's resonator.

The channel 230 is enclosed and, at least for the portion within the resonator, is constructed from a material that is substantially transparent at the wavelength of light generated by the gain medium. Thus for example in the case of a Krypton-Fluorine Excimer laser, the channel or a relevant portion of it may be made of fused quartz.

It will be appreciated that the channel may be shaped and have inlets and outlets suitable to provide desired flow characteristics, and that the channels in the figures are non-limiting examples for clarity only. However in an embodiment of the present invention, the walls of the channel at least within the resonator remain normal to the primary axis of the resonator.

Optionally, a wall of the channel may directly abut or comprise the high reflector itself. Similarly, a wall of the channel may directly abut or comprise a wall of the vessel containing the gain medium, or in the case of a solid gain medium, abut or comprise one end of the gain medium itself.

Figure 6 discloses decontamination apparatus 200A' with an alternative channel arrangement 230' in which the flow of air within the resonator is primarily in axis rather than transverse to it. The skilled person will appreciate that many configurations of channel may be considered within the scope of the invention, of which some examples are detailed below.

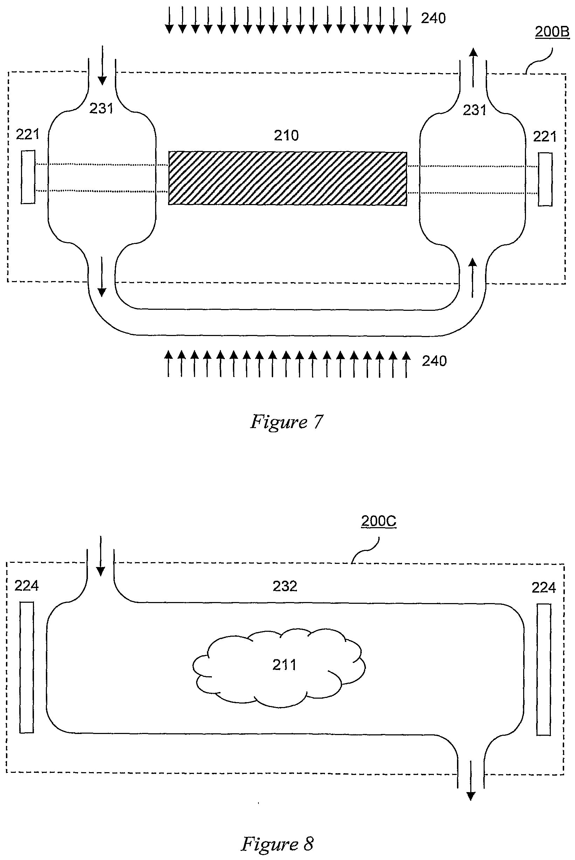

Referring now to Figure 7, in a second instance a decontamination apparatus 200B comprises a channel 231 that re-enters the resonator between the gain medium and the other reflector (shown on the right in Figure 7), thereby doubling the exposure of the fluid to the radiation in the resonator. Alternatively, a second parallel flow (not shown) may enter the resonator between the gain medium and the other reflector, thereby doubling the throughput of the fluid.

Figure 7 also illustrates an energy pump 240 for energising the gain medium, such as for example a flash-lamp, laser diode, electrical discharge, or RF or microwave energy source, as applicable to the type of gain medium used. It will be appreciated that such an energy pump may also have potential biocidal effects, such as sudden heat stress, electrocution, pulsed polarisation leading to structural stress, or the generation of by-products such as ozone.

Therefore, as seen in Figure 7, in an embodiment of the present invention channel 231 (or a separate, further channel or other fluid guiding means) may be arranged so as to pass the fluid for decontamination through a generated output (e.g. a plasma, electric field, electromagnetic radiation or ozone) of one or more parts of the energy pump. In the case where the energy pump generates biocidal by-products, optionally some or all of the energy pump may be placed within the flow of the fluid to enable the introduction of these by-products into the fluid, or where the energy pump is separated from the fluid, alternatively these by-products may be introduced into the fluid for example via a valve. Similarly, if a laser cooling system is used, the fluid may be thermally coupled to it to provide a further heat (or cold) shock.

Referring now to Figure 8, in a third instance a decontamination apparatus 200C comprises a channel 232 in lieu of a separate gain medium. The air for decontamination that is flowing through the channel then temporarily becomes the gain medium 211 for a nitrogen laser, utilising the nitrogen within the air itself. A nitrogen laser generates UV light at 337nm (near the so-called 'UVB' range). The pumping mechanism is not shown, but a mechanism suited to a conventional nitrogen laser may be used.

It will be appreciated that the second and third instances above are substantially similar to the first in other respects unless specified herein, including as appropriate the optional variants described previously.

Where the energy density within the resonator exceeds a desired minimum (for example, the energy density required to kill a certain class of microbes), then optionally further optics may be incorporated within the resonator.

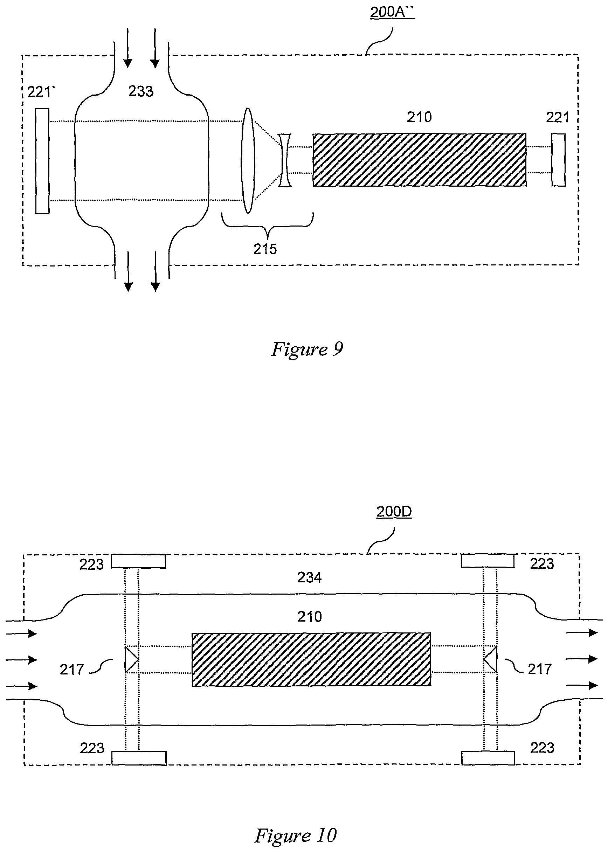

Now referring to Figure 9, a first such optic is a so-called beam expanding telescope 215 that may be incorporated within the first and second instances of the decontamination apparatus described previously. When disposed between the gain medium and the channel 233, the beam expanding telescope serves to increase the irradiated area of the channel for a corresponding reduction in energy density of laser light. Providing the energy density meets or exceeds the specified minimum, this enables a greater volume of air to be irradiated.

Now referring also to Figure 10, similarly a second such optic is a so-called beam splitter (for example a conical prism), which serves to deflect the resonator through 90 degrees and form a so-called light sheet, through which a comparatively large volume of air may flow. In this case, the high reflectors 223 are circumferential within a cylindrical body. Although two such light sheets are shown in figure 7 it will of course be appreciated that optionally only one may be used. The energy density of the light is a function of radius, and so the channel 234 may be designed accordingly to provide the minimum desired energy density at the maximum radius of the light sheet, given that the path length of the light still generates a standing wave.

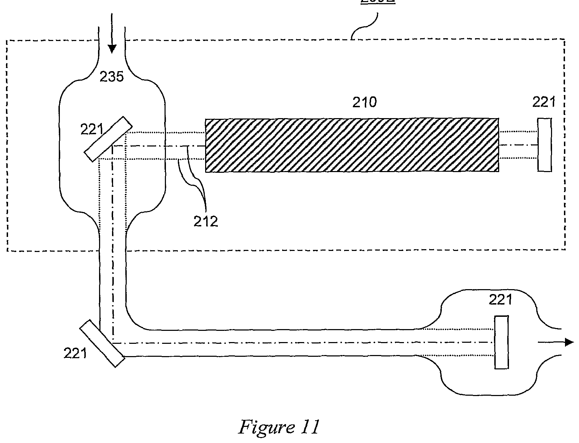

Now referring also to Figure 11, similarly a third such optic is the use of a suitably angled series of high reflectors to deflect the resonator into the axis of flow of the fluid for as long as is desired (given the generation of a standing wave and that the light remains above the minimum desired energy density). In this case successive reflections (for example with further mirrors similarly disposed) and possibly absorption by the fluid will attenuate the laser energy (whose path is highlighted in Figure 11 by dashed lines 212), but beneficially the air is exposed to the radiation for an extended period of time (or conversely much higher flows can be used). Other arrangements of reflectors to increase the dwell time of the fluid within the optical resonance cavity (or conversely to fold the optical resonance cavity a plurality of times within the region in which the fluid passes) will be apparent to the skilled person.

Consequently, alternatively or in addition one or more additional mirrors may be used to generate the optical resonance cavity in a more compact manner than a single linear space equal to the resonant path length. For example, the high reflectors bounding the resonant cavity may both be at one end of the laser, and a third mirror at the other end of the laser may be used to reflect the laser light from one high reflector back to the other, thereby effectively halving the physical length of the laser whilst preserving the full resonant cavity path length, and potentially exposing the fluid for decontamination to the optical cavity resonator twice instead of once. Other configurations will be apparent to a person skilled in the art.

It will be appreciated that other optical components known in the art may be similarly incorporated, including diffractive optics, structured light pattern generators and variable reflectivity mirrors.

It will also be appreciated that in addition to a stable optical resonance cavity, alternatively an unstable optical resonance cavity (where the beam size grows until it exceeds the area of a high reflector and is lost) may potentially be used.

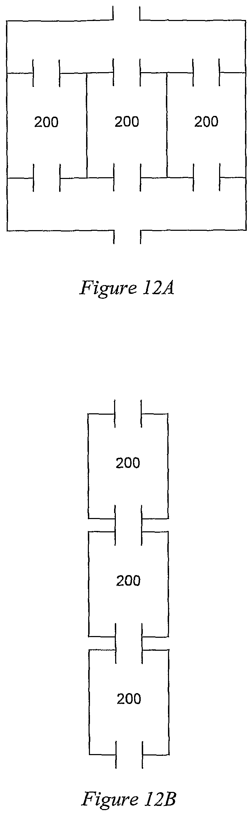

Referring now to Figures 12A and 12B, it will be appreciated that more than one decontamination apparatus may be used, either in parallel to improve throughput of the fluid as shown in Figure 12A, or in series to further improve decontamination levels in the fluid by additional rounds of exposure to radiation as shown in Figure 12B.

It will also be appreciated that in series different lasers could be used to address different contaminants of the fluid, and/or different versions of the contamination apparatus as described herein could be used. Moreover, multiple series of decontamination apparatuses may themselves be arranged in parallel (not shown).

Finally, the fluid may be recirculated or fed back into the system one or more times, thereby resulting in a plurality of passes through the decontamination system before being released or reintroduced back into the chamber 10.

Alternatively or in addition, for suitable fluids at least partial decontamination can be achieved by subjecting the fluid to a plasma discharge. Such discharges can be directly biocidal and can also (further) ionise the fluid. In the case where the fluid is air, it also produces ozone which as noted previously is also biocidal.

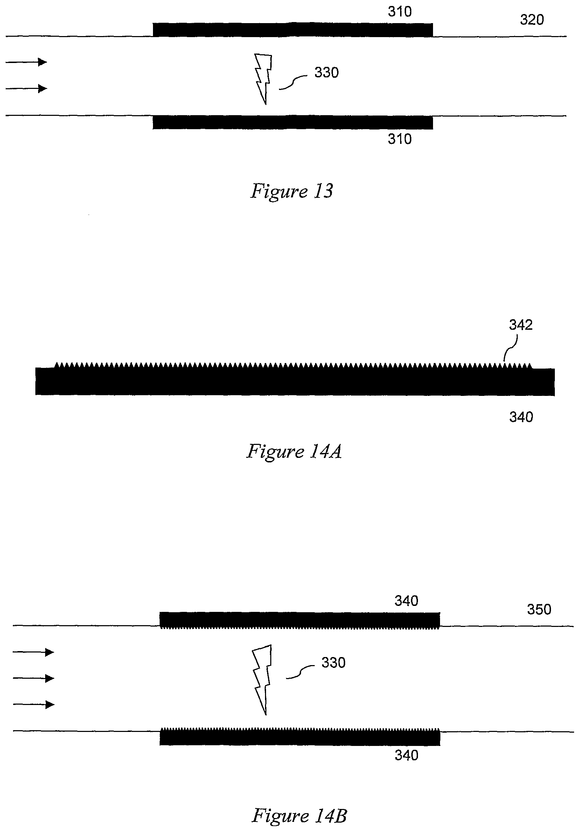

Referring to Figure 13, a cold plasma discharge can be achieved by placing two plate electrodes 310 on opposite sides of a (non-conductive) channel 320, through which the fluid is passed.

A high voltage potential is then applied across the plates. A so-called 'breakdown' discharge 330, in the form of a glow discharge, then occurs when ionised fluid conducts the electricity between the plates.

However, using conventional electrodes such discharges occur sporadically in random positions and at relatively high voltage potentials.

This is because for smooth electrode plates there is very little to differentiate possible discharge points on the surface, and so microscopic surface deviations and irregularities in composition become undesirably significant. Moreover, and of particular significance to their application for fluid decontamination, particulates in the fluid (for example dust motes, microbes, etc) that settle on the plates can also temporarily become point surface deviations and hence trigger a discharge. Clearly it is undesirable to be reliant on such unpredictable factors for plasma decontamination.

This also means it is difficult to ensure that substantially all of the fluid has been subjected to a plasma discharge. Furthermore, such smooth electrodes also need to be positioned relatively close together to achieve a breakdown discharge, thereby limiting fluid throughput.

A similar problem occurs where the electrode has one or more large surface features, such as a weld line or longitudinal peaks or furrows from a milling/rolling process used during manufacture, which can bias or limit the distribution of discharges across the width of the electrode (whilst potentially still leaving the longitudinal distribution unreliable) as described above).

Referring now also to Figure 14A, in an embodiment of the present invention one or preferably both of the electrode plates 340 are treated so as to have a surface 342 of substantially uniform roughness. This roughness can be expressed as a mean height variation in surface profile with respect to a mean distance across the surface. The uniformity can then be expressed as the variance or standard deviation about each mean or as a percentage of each mean. The degree of uniformity of roughness is achieved by applying a constraint to the permitted variance, standard deviation, or percentage.

This roughness provides a large number of discharge points over the working surface of each electrode (i.e. that portion of each electrode exposed to the fluid and located in opposition to a part of the other electrode) in a substantially uniform distribution. This therefore provides a substantially uniform distribution of potential discharge points, in contrast to the smooth electrodes and biased electrodes described previously.

The result is a more uniform plasma field between the electrodes and hence an improved exposure to the plasma and its possible products (such as ozone) by the fluid passing between the electrodes.

Referring now also to Figure 14B, a further advantage is that small peaks in the electrode surface facilitate breakdown discharge, allowing the electrical potential to be lower and/or for the electrodes to be placed further apart, thereby increasing the cross-sectional area of the duct 350 and hence the fluid flow rate.

Optionally, for example to adjust the properties of the electric field and/or the breakdown discharge, in addition to the fluid itself a further dielectric layer can be added between the electrodes. Typically this will be placed adjacent to one of the electrodes. Example dielectrics include ceramics such as alumina and barium titanate, though other suitable materials will be apparent to the person skilled in the art. Such a dielectric provides a substantially uniform source of preionisation, again improving the uniformity of discharge between the electrodes and reducing the likelihood that the discharge becomes an arc.

The surfaces of the electrodes can be roughened in a number of ways.

In an embodiment of the present invention, the electrodes are etched / ablated / marked using a laser. This provides control over the pitch and height of the peaks of the electrode surface, and if etched according to a pattern can also provide control over the distribution and density of potential discharge points.

A suitable laser may have the following properties:
- 1.: Power = 10W
- 2.: Pulse Repetition Frequecy = 20 Khz
- 3.: Energy per pulse = 10/(20 *10^3) = 0.5 mJ
- 4.: Lens Focal Length =160 mm
- 5.: Beam exit diameter = 5 mm
- 6.: Wavelength = 1064 nm
- 7.: Beam cutting Diameter = 1064 nm * 160 mm/5 mm = 34 um
- 8.: Energy Density = 0.5 mJ/(pi * (17 um)^2 = 0.55 MJoules /M^2

However, it will be appreciated that lasers with any suitable parameters may be selected; for example a laser with a higher energy density will allow for faster etching, whilst a laser with a narrower beam cutting diameter will allow for narrower peaks.

In an embodiment of the present invention, the beam is scanned over the electrode surface using a galvanometer system with two mirrors. Alternatively or in addition, the electrode could be moved under the laser beam.

The pattern generated on the electrode surface is then a function of, among others; laser power, pulse energy and frequency, laser wavelength, the melting point of the electrode material and its specific heat capacity, and net scanning velocity and direction. Such parameters may be computer controlled during the etching process.

Alternatively or in addition to the above etching technique, the electrodes can be acid-etched using a protective pattern on the electrode surface (for example photolithography) to define the distribution of peaks and troughs with a similar control of their properties.

Alternatively or in addition the electrode surface may be randomly marked by the use of abrasive particles of a suitable size.

An alternative to making the surface rough by etching or abrasion is to make it rough by deposition. In addition to conventional deposition methods such as sputter deposition, known and suitably controlled methods of sintering, cold-pressing of metal particles, crystallisation, or electrolysis may be used on the electrode to generate a rough surface. Other techniques will be apparent to a person skilled in the art.

As an alternative to the addition or depletion of electrode material, the electrode may be cast to have a rough pattern, or stamped with a rough pattern.

Given the above options, it will therefore be appreciated that within a chosen mean and variance range, the peaks on the rough surface may be substantially random or, where the method of manufacture enables it, form a repeating or non-repeating pattern, itself either regular, irregular or pseudo-random. It will also be appreciated that various methods may be either particularly suitable or unsuitable to different granularities of roughness and so may be selected as applicable.

The scale or granularity of the roughness can depend upon the application. Efficient electron emission is possible from metallic and other conductive peaks in the order of several hundred nanometres tall, as defined from base/valley to peak (although a suitable laser can generate surface features in the order of 20nm or less; see "Surface nanostructuring of metals by laser irradiation: effects of pulse duration, wavelength and gas atmosphere" by A. Pereira Et. Al., Appl. Phys. A 79, 1433-1437 (2004)).

Such electron emission peaks may be used for example for the decontamination (or rendering inert) of chemical contaminants in a fluid. For systems aimed at destroying particulates such as viruses and microbes, then a surface roughness with a vertical extent in the order of the diameter of the particles themselves mitigates against them becoming a major source of discharge points on the electrode surface as discussed previously.

Viruses are typically 50 to 200 nanometers long and so will tend to be smaller than, or of similar size to, the peaks described above.

Bacteria are typically from 1 to 10 micrometres (microns) in diameter, though some are hundreds of micrometres across.

Likewise, dust varies in diameter, but is typically between 10 and 100 micrometers in diameter. Dust mites are typically between 200-400 micrometers in length, whilst their eggs are around 15-20 micrometers in diameter.

Thus for different applications, the granularity of roughness chosen may vary between around 200 nanometers to around 500 micrometres, or by four orders of magnitude. For general applications where pollutants may include microbes and dust, the granularity of roughness chosen may vary between around 1 micrometer and 200 micrometers. For applications aimed specifically at microbes, the granularity of roughness chosen may vary between around 1 micrometer and 20 micrometers, unless a particular target microbe is known to be significantly larger.

Typically, the pitch or wavelength of the peaks (i.e. their mean separation) will be of a similar order to their height, though this is not a strict requirement.

In an embodiment of the present invention, alternatively the roughness may be chosen in practice in response to the minimum particulate size for which a filter upstream in the system is rated. Thus for example a filter rated to remove particles larger than 100µm may be complemented by plasma electrodes with a roughness granularity in the order of 100µm or slightly larger.

In another embodiment of the present invention, alternatively the roughness may be chosen in practice to induce boundary layer turbulence in the fluid, thereby improving the mixing of biocidal compounds generated by the plasma discharge (for example ozone, if the fluid is air) and redistribution of contaminants in the fluid as it passes through the plasma, again improving uniformity of exposure to the plasma. A typical roughness in this embodiment would be in the order of 1000 micrometers.

A common measurement of roughness is called *Ra* or *Rq,* and is typically measured as the root-mean-square (RMS) of the vertical profile of a surface over a region of evaluation. As non-limiting examples, Table 1 overleaf gives the *Ra* value for the roughness of some possible electrode materials as a function of laser etching traversal for a laser with properties as given previously herein.

**Table 1. Example roughness measures of electrodes**

| Etching Laser traversal, mm/s | Copper Ra[µm] | Stainless steel Ra[µm] | Brass Ra[µm] | Aluminium Ra[µm] |
|---|---|---|---|---|
| 200 | 0.3122 | 0.4936 | 0.5114 | 1.2120 |
| 50 | 0.8641 | 1.2415 | 1.2874 | 2.7596 |
| 10 | 6.7255 | 9.4289 | 4.2825 | 8.0201 |

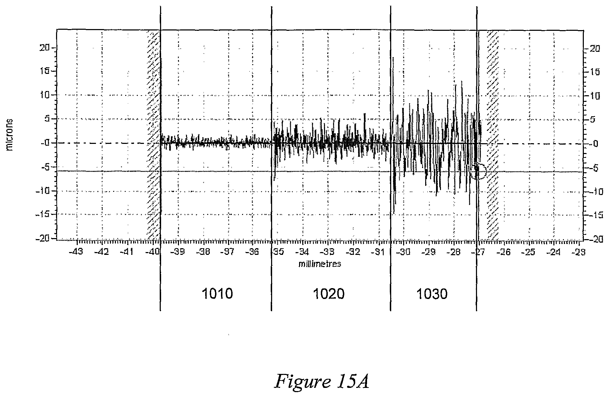

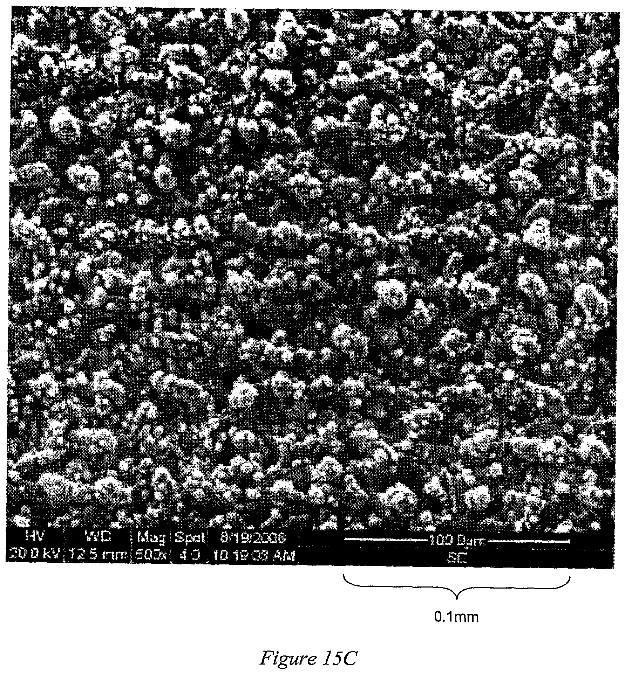

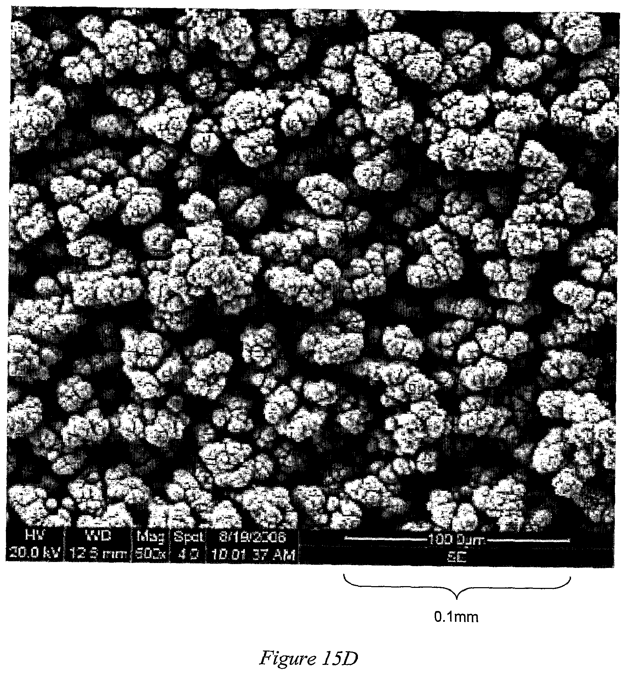

It will be appreciated that for each case, faster laser traversal or a lower laser energy density would reduce the roughness, whilst slower laser traversal or a higher energy density would increase the roughness achieved. Note that whilst, for example, the RMS roughness of the Brass at 10mm/s etching is approximately 4 Ra[µm], the absolute peak-to-trough granularity of roughness in will be in the order of 15 µm, as can be seen in Figure 15A.

Figure 15A shows the physical measurements on a brass test surface used to generate the Ra numbers for brass given above. In this figure, the x-axis denotes millimetres traversed across the surface and the y-axis is the measured vertical profile of the surface in micrometres. Sections 1010, 1020 and 1030 correspond to laser traversal speeds of 200, 50 and 10 mm/s respectively. It can be seen that there is a characteristic surface roughness for each traversal condition, and that the variance in peak height in each successive section is such that very few peaks in the first section 1010 are as high as the mean peak height in the section 1020, for which in turn there are very few peaks that are as high as the mean peak height in the third section 1030.

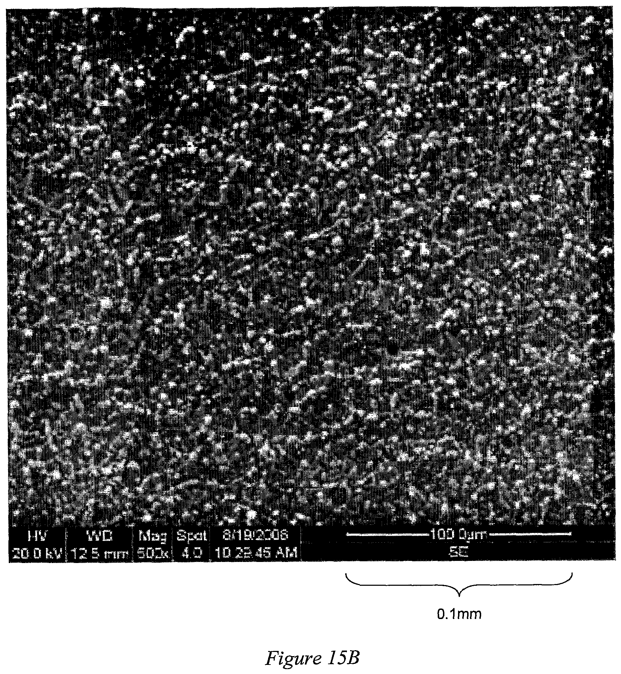

Referring now to Figures 15B-D, images of the brass surface in sections 1010, 1020 and 1030 are shown at the same scale (0.1mm being indicated on each figure), demonstrating both the clear difference in surface granularity between each section and also the clear uniformity of granularity and spatial distribution within each section.

The typical absolute peak-to-trough granularity of roughness at 10mm/s etching for the other metals in Table 1 are approximately 40 µm for Aluminium (having a relatively low melting point and specific heat capacity), 25 µm for Copper, and 50 µm for Stainless Steel.

At any given granularity of roughness, the variance about the mean of the roughness of granularity affects whether certain peaks become frequent sources of discharge and hence reduce homogeneity.

As a non-limiting example, a possible requirement or constraint on variance is that less than a threshold percentage (e.g. five percent) of peaks should exceed one standard deviation from the mean peak height. Alternatively, a constraint on variance may be absolute. As a non-limiting example, a threshold percentage (e.g. five percent) of peaks should remain within a peak height distribution of 10 microns about the mean peak height. Clearly in this case the absolute value chosen may differ according to the desired mean peak height, and potentially also according to the material property of the electrode, the type of marking process, properties of any additional dielectric used, etc, and may be determined empirically for the specific electrode and/or electrode deployment chosen.

Such constraints may be enforced by rejecting those electrodes that do not satisfy them.

In addition, the constraint may be applied locally by taking a local mean of peak height, for example within a 1, 5 or 10mm square sample area (the area chosen may depend upon the global mean peak height and the peak pitch).

It should be noted however that if occasional relatively high peaks are initially favoured as discharge points, they will wear down (for example by the well-known mechanism of sputtering) until they are of a similar height to the majority of peaks; hence this problem is to an extent self-regulating over the lifetime of the electrodes.

Likewise a similar type of constraint (though potentially using a different choice tolerance) may be applied to the variability of peak pitch or wavelength, i.e. mean peak separation. Notably the possible mean peak separation may vary from approximately the same as mean peak height, if the peaks are densely packed, to a much greater mean separation distance if the peaks are comparatively sparse. Therefore, if assessing the uniformity of the peaks, the sample area for evaluation should be a function of mean separation. In general, however, a local region of a roughened electrode surface will have a substantially continuously varying profile between peaks. The tolerance in uniformity of peak separation can be empirically determined for the chosen type and deployment of electrode.

In the case of roughening using laser etching, the minimum peak separation will be a function of the beam cutting width; for example, the separation may be ½ or ¼ of the beam cutting width. For other marking methods, the minimum peak separation will similarly be a function of the technique used.

It will be appreciated that for some specified mean peak heights it may be necessary to smooth or polish the electrode as a preliminary step in order to achieve an initial surface profile whose random or sporadic deviations are less than the target mean peak height.

The issue of wear on electrodes, including those described herein (for example by sputtering, as noted previously) is significant, since whilst the electrodes themselves are intended to be easy to replace (for example being placed in an electrode holder that plugs into a high voltage rail), in practice replacement may be a non-trivial exercise - for example, the fluid conditioning system may be in a hard-to-access part of a building or installation, and the removal and replacement of the electrodes may require a mechanism to isolate the plasma decontamination portion by diverting fluid flow elsewhere, or require the shutting down of the fluid conditioning system. In addition, servicing may compromise seals on the equipment, allowing fluids either in or out, or depending on the fluids concerned may be hazardous to maintenance personnel.

To address this, in an embodiment of the present invention the potential between the electrodes is set at a level insufficient to generate plasma, but sufficient to polarise bacteria and the like within the resulting electric field. Avoiding discharges extends the life of the electrodes, whilst polarisation physically distorts bacteria by effectively pulling on them between the electrodes. This distortion can itself kill the bacteria, or it can weaken the bacteria structurally so that subsequent exposure to UV radiation in the laser system described previously is even more effective.

Optionally, in this non-plasma embodiment, the electric field can be rapidly varied so as to induce multiple stresses upon microbial contaminants as they pass through it, thereby increasing damage to them. This may be achieved by variations at a voltage controller, not shown (e.g. a square wave, sinusoid or saw-tooth voltage profile). The frequency or frequencies of variation can optionally be tuned to resonant frequencies of a microbe's body; such frequencies can be determined empirically for common species.

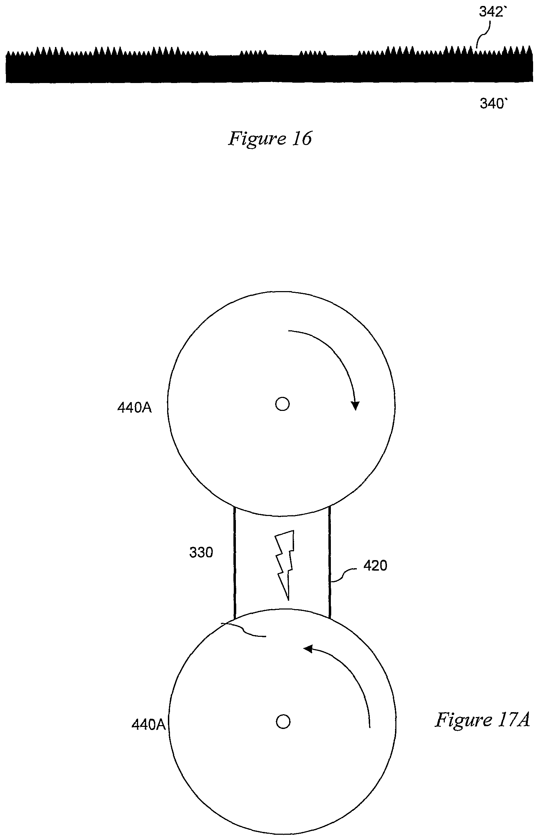

Referring to Figure 16, alternatively or in addition, the electric field can optionally be varied by changing the granularity of roughness 342' over the length of the electrodes 340'. Thus for example a first local region of the electrode has a first granularity of roughness conforming to a first constraint on variability, whilst a second region of the electrode has a second granularity of roughness, optionally conforming to a section constraint on variability. Thus whilst the roughness of the electrodes remains locally substantially uniform, globally (typically longitudinally) it varies so that a microbe passing along the duct is exposed to a varying field. Optionally, some regions of the electrode may remain smooth (i.e. not roughened).

It will be appreciated that a locally substantially uniform but globally variable electrode roughness may also be used in combination with plasma discharges; here the plasma discharges will show improved homogeneity locally, but will vary, along with the electric field, across the extent of the electrodes in response to the roughness profile.

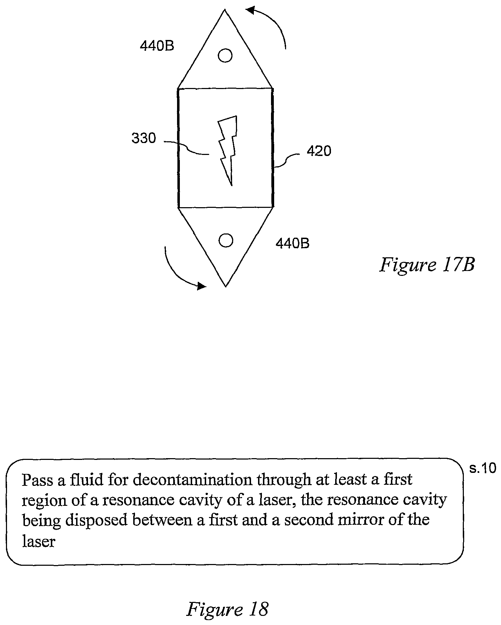

Referring now to Figures 17 A and B, alternatively or in addition in an embodiment of the present invention wear on electrodes and the frequency of replacement or maintenance can be mitigated by making one or preferably both of the electrodes rotatable, so as to make a new surface or portion thereof the working surface of the electrode as a function of time.

Figure 17A shows a first instance in which the electrodes 440A are cylindrical; such electrodes may for example rotate continuously or periodically (e.g. by 1 degree per unit time interval), thereby over time distributing wear and tear over an electrode surface much larger than the current working surface. The interface between the electrode and the duct 420 may comprise a resilient seal (not shown), and/or the electrode may be enclosed within a sealed cavity (not shown). Such a cavity may be flushable.

It will be appreciated that the electrode need not be a full cylinder, but may be arcuate and rotate back and forth.

In conditions where the curvature of such an electrode is undesirable, the electrode 440B may be polygonal, as seen in Figure 17B. In this case, the faces of the polygon act in turn as the (flat) working surface of the electrode. Again the interface between the electrode and the duct may comprise a resilient seal (not shown), primarily of use when the polygon is in an operational configuration, and/or the electrode may be enclosed within a sealed cavity, primarily of use when the polygon is rotating between faces and hence temporarily not forming an interface with the duct. Such a cavity may then be flushable.

In the case of a polygonal rotatable electrode, the interval between rotations will generally by much greater as the electrode will not function normally during the rotation process. Thus, for example, the rotation may occur during a scheduled maintenance period, or preceding start up of the conditioning unit pump 110 or following shutdown of the pump. Alternatively or in addition, the electrode may rotate to retire a face when it is detected that the face has worn down to an unacceptable level; this may be detected in the plasma discharge embodiments described above, for example, by whether the mean potential required to generate a plasma has exceeded a threshold level, indicative that the rough surface of that face of the electrode has worn down due to sputtering or other damage.

It will be appreciated that rotatable electrodes are applicable not just to the systems described herein, but to any system using electrode plates or similar that require maintenance or replacement, including conventional smooth electrodes.

It will also be appreciated that potentially only one of a pair or set of electrodes may be rotatable; for example the electrode on a more inaccessible side of a duct within an installation.

The above decontamination arrangement of static or rotatable electrodes, providing a homogenous plasma field or a variable electric field or a combination of the two, can operate independently or can be placed either upstream or downstream of the laser arrangement described herein. However, placing the electrode arrangement upstream (and preferably immediately upstream) of the laser arrangement provides a synergy between the two, as the stresses placed upon surviving biological structures by the plasma and/or electric field increases the likelihood of subsequent destruction of such structures by the laser radiation.

It will be appreciated that such electrode arrangements can operate in parallel ducts to increase fluid throughput, and/or electrode arrangements with, for example, different field characteristics can be placed successively within the flow of the fluid.

In addition, it will be appreciated by a person skilled in the art that an electrode arrangement such as described herein can also be used for laser discharge electrodes.

Referring now to Figure 18, a method of fluid decontamination using a laser, wherein the laser has a resonance cavity, comprises the step of:
passing (s.10) a fluid for decontamination through at least a first region of the resonance cavity.

It will be apparent to a person skilled in the art that variations in the above method corresponding to operation of the various embodiments of the apparatus described above are considered within the scope of the present invention, including but not limited to:
- The fluid flowing within an enclosed channel substantially transparent to the emission frequency of the laser;
- The laser comprising a beam expanding telescope;
- The laser comprising a light sheet apparatus;
- The fluid acting as the gain medium as it passes through the resonance cavity;
- The laser being any suitable laser, including an excimer laser or nitrogen laser operating at UV frequencies, or a carbon-dioxide laser operating at an infra-red frequency
- The laser system being complemented by an electrode arrangement in which at least a first portion of the electrode has a uniform degree of roughness;
- The laser system being complemented by a rotateable electrode arrangement;
- The laser system being complemented by an electrode arrangement that generates a fluctuating electric field; and
- The laser system being complemented by an electrode arrangement that generates a substantially homogenous plasma field.

Finally, it will be appreciated that some of the pattern generation methods disclosed herein may be carried out on conventional hardware suitably adapted as applicable by software instruction or by the inclusion or substitution of dedicated hardware. For example, the laser etching and sputter deposition techniques may be computer-controlled to generate a surface pattern comprising changes made to the surface profile, wherein the changes in surface profile satisfy a constraint on the variability of said changes made to the surface profile.

Thus the required adaptation to existing parts of a conventional equivalent surface modifying device may be implemented in the form of a computer program product comprising processor implementable instructions stored on a data carrier such as a floppy disk, optical disk, hard disk, PROM, RAM, flash memory or any combination of these or other storage media, or transmitted via data signals on a network such as an Ethernet, a wireless network, the Internet, or any combination of these of other networks, or realised in hardware as an ASIC (application specific integrated circuit) or an FPGA (field programmable gate array) or other configurable circuit suitable to use in adapting the conventional equivalent device.

## Claims

1. A fluid decontamination apparatus (200A) comprising a laser, the laser having an optical resonance cavity disposed between a first reflector (221) and a second reflector (221), and comprising means to pass a fluid for decontamination through at least a first region of the optical resonance cavity, thereby in operation exposing the fluid to a decontaminating effect of the radiation within the optical resonance cavity, **characterised in that**
the decontamination apparatus further comprises:
an energy pump (240) for stimulation of the laser; and
means to pass the fluid for decontamination through at least a first region of a generated output of the energy pump.

2. A decontamination apparatus according to claim 1 in which the fluid for decontamination flows within an enclosed channel (230) passing through the at least first region of the optical resonance cavity, and in which a portion of the enclosed channel on a main axis of optical resonance (212) is substantially transparent at the laser frequency.

3. A decontamination apparatus according to any one of the preceding claims, in which the optical resonance cavity comprises a beam expanding telescope (215), arranged to expand the effective volume of the first region of the resonance cavity.

4. A decontamination apparatus according to any one of the preceding claims, comprising a prism (217) disposed to form a light sheet as part of the optical resonance cavity, and in which the fluid is arranged to pass through the light sheet.

5. A decontamination apparatus according to any one of claims 1 to 4, comprising an electric field generator through which the fluid for decontamination flows;
in which at least a first electrode (310) of the electric field generator is rotatable about an axis so as to change a surface region (342) of the electrode acting as the available working surface of the electrode.

6. A decontamination apparatus according to claim 5, in which the electric field is arranged to generate plasma discharges.

7. A fluid conditioning system comprising the decontamination apparatus of any one of the preceding claims.

8. A method of fluid decontamination using a laser, the laser having an optical resonance cavity disposed between a first reflector (221) and a second reflector (221), the method comprising the step of passing a fluid for decontamination through at least a first region of the optical resonance cavity, thereby exposing the fluid to a decontaminating effect of the radiation within the optical resonance cavity.

9. A method of fluid decontamination according to claim 8, comprising the step of passing the fluid for decontamination through the at least first region of the optical resonance cavity in an enclosed channel (230) having a portion on a main axis of optical resonance (212) being substantially transparent at the laser frequency.

10. A method of fluid decontamination according to claim 8 or claim 9, in which at least a first component of the fluid for decontamination acts as a gain medium (211) whilst it is passing through the at least first region of the optical resonance cavity.

11. A method of fluid decontamination according to claim 10 in which the fluid is air and the at least first component is nitrogen.

12. A method of fluid decontamination according to any one of claims 8 to 11, comprising the step of:
passing the fluid for decontamination through an electric field generated using electrodes (310);
in which at least one of the electrodes (310) is rotatable about an axis so as to change a surface region (342) of the electrode acting as the available working surface of the electrode.

13. A method of fluid decontamination according to claim 12, in which the electric field generates plasma discharges.

## Patentansprüche

1. Fluiddekontaminierungsvorrichtung (200A), welche einen Laser umfasst, wobei der Laser einen optischen Resonator aufweist, welcher zwischen einem ersten Reflektor (221) und einem zweiten Reflektor (221) angeordnet ist, und welche Mittel umfasst, um ein Fluid zur Dekontaminierung durch zumindest eine erste Region des optischen Resonators zu leiten, wodurch das Fluid während des Betriebs einer dekontaminierenden Wirkung der Strahlung innerhalb des optischen Resonators ausgesetzt wird,
**dadurch gekennzeichnet, dass**
die Dekontaminierungsvorrichtung ferner Folgendes umfasst:
eine Energiepumpe (240) zur Anregung des Lasers; sowie
Mittel, um das Fluid zur Dekontaminierung durch zumindest eine erste Region einer erzeugten Ausgangsleistung der Energiepumpe zu leiten.

2. Dekontaminierungsvorrichtung nach Anspruch 1, bei welcher das Fluid zur Dekontaminierung innerhalb eines geschlossenen Kanals (230) strömt, welcher durch die zumindest erste Region des optischen Resonators führt, und bei welcher ein Abschnitt des geschlossenen Kanals auf einer optischen Hauptresonanzachse (212) für die Laserfrequenz im Wesentlichen durchlässig ist.

3. Dekontaminierungsvorrichtung nach einem der vorangehenden Ansprüche, bei welcher der optische Resonator ein strahlaufweitendes Teleskop (215) umfasst, welches angeordnet ist, um das effektive Volumen der ersten Region des Resonators zu erweitern.

4. Dekontaminierungsvorrichtung nach einem der vorangehenden Ansprüche, welche ein Prisma (217) umfasst, das angeordnet ist, um eine Lichtwand als Teil des optischen Resonators auszubilden, und bei welcher das Fluid angeordnet ist, um die Lichtwand zu durchlaufen.

5. Dekontaminierungsvorrichtung nach einem der Ansprüche 1 bis 4, welche einen elektrischen Feldgenerator umfasst, durch welchen das Fluid zur Dekontaminierung strömt;
bei welcher zumindest eine erste Elektrode (310) des elektrischen Feldgenerators um eine Achse drehbar ist, um eine Oberflächenregion (342) der Elektrode zu ändern, welche als die verfügbare Arbeitsoberfläche der Elektrode fungiert.

6. Dekontaminierungsvorrichtung nach Anspruch 5, bei welcher das elektrische Feld angeordnet ist, um Plasmaentladungen zu erzeugen.

7. Fluidaufbereitungssystem, welches die Dekontaminierungsvorrichtung nach einem der vorangehenden Ansprüche umfasst.

8. Verfahren zur Fluiddekontaminierung unter Verwendung eines Lasers, wobei der Laser einen optischen Resonator aufweist, welcher zwischen einem ersten Reflektor (221) und einem zweiten Reflektor (221) angeordnet ist, wobei das Verfahren den Schritt umfasst, ein Fluid zur Dekontaminierung durch zumindest eine erste Region des optischen Resonators zu leiten, wodurch das Fluid einer dekontaminierenden Wirkung der Strahlung innerhalb des optischen Resonators ausgesetzt wird.

9. Verfahren zur Fluiddekontaminierung nach Anspruch 8, welches den Schritt umfasst, das Fluid zur Dekontaminierung in einem geschlossenen Kanal (230), welcher einen Abschnitt auf einer optischen Hauptresonanzachse (212) aufweist, der für die Laserfrequenz im Wesentlichen durchlässig ist, durch die zumindest erste Region des optischen Resonators zu leiten.

10. Verfahren zur Fluiddekontaminierung nach Anspruch 8 oder Anspruch 9, bei welchem zumindest ein erster Bestandteil des Fluids zur Dekontaminierung als Verstärkungsmedium (211) fungiert, während dieses die zumindest erste Region des optischen Resonators durchläuft.

11. Verfahren zur Fluiddekontaminierung nach Anspruch 10, bei welchem das Fluid Luft ist und der zumindest erste Bestandteil Stickstoff ist.

12. Verfahren zur Fluiddekontaminierung nach einem der Ansprüche 8 bis 11, welches den folgenden Schritt umfasst:
Leiten des Fluids zur Dekontaminierung durch ein elektrisches Feld, welches unter Verwendung von Elektroden (310) erzeugt wird;
wobei zumindest eine der Elektroden (310) um eine Achse drehbar ist, um eine Oberflächenregion (342) der Elektrode, welche als die verfügbare Arbeitsoberfläche der Elektrode fungiert, zu ändern.

13. Verfahren zur Fluiddekontaminierung nach Anspruch 12, bei welchem das elektrische Feld Plasmaentladungen erzeugt.

## Revendications

1. Dispositif de décontamination de fluide (220A) comprenant un laser, le laser ayant une cavité de résonance optique disposée entre un premier réflecteur (221) et un second réflecteur (221), et comprenant des moyens pour faire passer un fluide destiné à une décontamination à travers au moins une première zone de la cavité de résonance optique, en exposant ainsi en fonctionnement le fluide à un effet de décontamination du rayonnement à l'intérieur de la cavité de résonance optique,
**caractérisé en ce que**
le dispositif de décontamination comporte en outre :
une pompe d'énergie (240) pour stimulation du laser ; et
des moyens pour faire passer le fluide destiné à une décontamination à travers au moins une première zone d'une sortie générée de la pompe d'énergie.

2. Dispositif de décontamination selon la revendication 1, dans lequel le fluide destiné à une décontamination s'écoule dans un canal renfermé (230) passant à travers la au moins première zone de la cavité de résonance optique, et dans lequel une partie du canal renfermé sur un axe principal de résonance optique (212) est sensiblement transparente à la fréquence de laser.

3. Dispositif de décontamination selon l'une quelconque des revendications précédentes, dans lequel la cavité de résonance optique comprend un télescope de dilatation de faisceau (215), agencé pour agrandir le volume effectif de la première zone de la cavité de résonance.

4. Dispositif de décontamination selon l'une quelconque des revendications précédentes, comprenant un prisme (17) disposé pour former une feuille légère en tant que partie de la cavité de résonance optique, et dans lequel le fluide est agencé pour passer à travers la feuille légère.

5. Dispositif de décontamination selon l'une quelconque des revendications 1 à 4, comprenant un générateur de champ électrique à travers lequel le fluide destiné à une décontamination s'écoule ;
dans lequel au moins une première électrode (310) du générateur de champ électrique peut tourner autour d'un axe de manière à changer une zone de surface (342) de l'électrode, agissant comme surface d'usinage disponible de l'électrode.

6. Dispositif de décontamination selon la revendication 5, dans lequel le champ électrique est agencé pour générer des décharges de plasma.

7. Système de conditionnement de fluide comprenant le dispositif de décontamination selon l'une quelconque des revendications précédentes.

8. Procédé de décontamination de fluide en utilisant un laser, le laser ayant une cavité de résonance optique disposée entre un premier réflecteur (221) et un second réflecteur (221), le procédé comprenant l'étape consistant à faire passer un fluide destiné à une décontamination à travers au moins une première zone de la cavité de résonance optique, en exposant ainsi le fluide à un effet de décontamination du rayonnement à l'intérieur de la cavité de résonance optique.

9. Procédé de décontamination de fluide selon la revendication 8, comprenant l'étape consistant à faire passer le fluide destiné à une décontamination à travers la au moins une première zone de la cavité de résonance optique dans un canal renfermé (230) ayant une partie sur un axe principal de résonance optique (212) sensiblement transparente à la fréquence de laser.

10. Procédé de décontamination de fluide selon la revendication 8 ou 9, dans lequel au moins un premier composant du fluide destiné à une décontamination agit comme milieu de gain (211) tout en passant à travers la au moins une première zone de la cavité de résonance optique.

11. Procédé de décontamination de fluide selon la revendication 10, dans lequel le fluide est de l'air, et le au moins un premier composant est de l'azote.

12. Procédé de décontamination de fluide selon l'une quelconque des revendications 8 à 11, comprenant l'étape consistant à :
faire passer le fluide destiné à une décontamination à travers un champ électrique généré en utilisant des électrodes (310) ;
dans lequel au moins une des électrodes (310) peut tourner autour d'un axe de manière à changer une zone de surface (342) de l'électrode agissant comme surface d'usinage disponible de l'électrode.

13. Procédé de décontamination de fluide selon la revendication 12, dans lequel le champ électrique génère des décharges de plasma.
